# EUROPEAN PATENT SPECIFICATION

(11) **EP 3 570 935 B1**
(45) Date of publication and mention of the grant of the patent: **12.08.2020**
(21) Application number: 18703404.6
(22) Date of filing: 15.01.2018
(51) Int. Cl.: A61N 1/36

(54) **SYSTEM FOR PROVIDING THERAPY TO A PATIENT VIA APPLICATION OF A BROAD SPECTRUM OF TUNABLE ELECTRICAL NOISE SIGNALS**
SYSTEM ZUR BEHANDLUNG EINES PATIENTEN DURCH ANWENDUNG EINES BREITSPEKTRUMS VON ABSTIMMBAREN ELEKTRISCHEN RAUSCHSIGNALEN
SYSTÈME POUR FOURNIR UNE THÉRAPIE À UN PATIENT PAR APPLICATION D'UN LARGE SPECTRE DE SIGNAUX DE BRUIT ÉLECTRIQUE ACCORDABLES

(30) Priority: 18.01.2017 US 201762447504 P
(43) Date of publication of application: 27.11.2019
(73) Proprietor: Soin Neuroscience, LLC, Dayton OH 45458 (US)
(72) Inventor: SCHEPIS, Eric A., Alpharetta Georgia 30004 (US)
(74) Representative: Eisenführ Speiser
(86) International application number: PCT/US2018/013700
(87) International publication number: WO 2018/136354

(56) References cited:
- EP-A1- 2 703 042
- WO-A1-93/18821

## Description

### FIELD OF THE INVENTION

The present invention generally relates to a system for providing therapy to a patient via the application of a tunable electrical noise signal.

### BACKGROUND OF THE INVENTION

Periodic electrical waveforms are commonly used to stimulate nervous tissue to treat patients with neurological disorders. Fourier's theorem teaches that periodic waveforms are composed of sinusoidal signals that are harmonically related to the repetition frequency of the original signal. "Harmonically related" means that the frequency of the sinusoids is an integral multiple of some "basic" or "fundamental" number. That is, the frequency is one times, two times, three times, etc. the basic or fundamental number. Each of the component frequencies is known as a harmonic, and, collectively, these component frequencies are known as the Fourier series. The amplitude of each harmonic is correlated to the amplitude of the fundamental frequency.

Altogether, the electrical stimulation waveforms that are used today do not enable the user to modulate stimulation energy in harmonics independently of the fundamental frequencies, and the stimulation frequencies are confined to the harmonics within the original signal, excluding the frequencies in between. For example, periodic biphasic square-wave pulses are used to stimulate nervous tissue to treat pain, motor, and sensory disorders. The Fourier series of a biphasic square-wave pulse includes the fundamental frequency, and its odd multiples (i.e., it does not have even numbered harmonics). The amplitude of each harmonic is represented as 1/integral multiple of the fundamental frequency's (i.e. 3, 5, 7, 9) amplitude. That is, constant-voltage, biphasic square-wave pulses delivered at 200 Hertz (Hz) and 1 volt (V), has a fundamental frequency (amplitude) of 200 Hz (1/1 V), and harmonics at 600 Hz (1/3 V)), 1000 Hz (1/5 V), 1400 Hz (1/7 V), and 1800 Hz (1/9 V), etc. The biphasic square-wave pulse does not allow the user to modulate the energy of each harmonic independently of the energy within the fundamental frequency, and the stimulation frequencies are confined to its odd integral harmonics.

An electrical stimulation waveform that is flexible in both frequency spectrum and the energy content of each frequency band would be better enabled to accommodate for patient variability and disease state, ultimately leading to better patient outcomes. Untuned electrical noise has been used to modulate the excitation of neural tissues, but it has not been used to optimize neural and non-neural activity to treat disease. As such, there is an unmet need for a method and system for delivering a broad spectrum of electrical noise signals to neural tissue, non-neural tissue, or a combination thereof (e.g., tissue within or adjacent the brain, the spinal cord, a dorsal root ganglion, a sympathetic chain ganglion, a peripheral nerve, etc.) of a patient, where the electrical noise signals are tunable. A broad spectrum of electrical noise signals would enable improved modulation of the target neural tissue, non-neural tissue, or a combination thereof, and the tunability feature would account for disease and patient variability, where feedback from the patient could be used to tune or adjust the broad spectrum of electrical noise signals delivered to the patient.

WO 93/18821 relates to a physiological monitoring system for monitoring the condition of a patient's body tissue. The device includes electrodes for contacting the tissue to be monitored, circuitry for generating electrical signals at at least two frequencies for application to the tissue and circuitry for monitoring the impedance of the tissue, at the frequencies applied. The monitoring apparatus may be practiced in the context of an implantable stimulator, such as a cardiac pacemaker, in which the pulse frequency is varied as a function of the detected condition of the tissue.

EP 2 703 042 A1 relates to a radiofrequency hyperthermia device for personalized treatment and diagnosis using capacitive coupling and without dipole antenna comprising a radiofrequency source, an amplifier, a sensor and a modulation signal input/generator. The radiofrequency source produces a source signal which is modulated by the modulation signal input/generator using the phase information generated by homeostasis of the target to generate a modulated source signal. The modulated source signal is amplified by the amplifier and directed to a target, and the sensor detects the phase information generated by homeostasis of the target to provide a feedback signal, wherein the feedback signal modulates the source signal to generate a personalized modulated signal.

### SUMMARY OF THE INVENTION

The invention is defined in the appended claims. Aspects, embodiments and examples disclosed herein which do not fall within the scope of the appended claims do not form part of the invention, and are merely provided for illustrative purposes.

The problems described above are addressed by the present invention, which encompasses systems for delivering a broad spectrum of electrical noise signals to target neural tissue, non-neural tissue, or a combination thereof, where the signals are tunable.

The present invention suggests a system for providing therapy to a patient. The system includes an electrode; a noise generator coupled to the electrode; and a controller; wherein the controller instructs the noise generator to deliver a broad spectrum of electrical noise signals to target neural tissue, non-neural tissue, or a combination thereof via the electrode, wherein the broad spectrum of electrical noise signals are tunable, and wherein the controller is configured to tune the broad spectrum of electrical noise signals to optimize the therapy provided to the patient based on feedback received from the patient.

In one embodiment, the controller can tune the broad spectrum of electrical noise signals by adjusting energy contained within a frequency band.

In still another embodiment, the controller can tune the broad spectrum of electrical noise signals by adjusting a phase component of the broad spectrum of electrical noise signals.

In yet another embodiment, the therapy provided to the patient can treat pain.

In one more embodiment, the therapy provided to the patient can treat an autonomic disorder.

In an additional embodiment, the therapy provided to the patient can treat a sensory disorder.

In another embodiment, the therapy provided to the patient can treat a motor disorder.

In one particular embodiment, the therapy provided to the patient can elicit plastic changes in neural tissue, non-neural tissue, or a combination thereof to mitigate or abolish a pathophysiologic disease or syndrome.

In still another embodiment, the target neural tissue, non-neural tissue, or a combination thereof can be located within or adjacent the patient's brain, the patient's spinal cord, a dorsal root ganglion, a sympathetic chain ganglion, or a peripheral nerve.

In yet another embodiment, the electrode can be percutaneous, transcutaneous, or implantable.

In one more embodiment, the noise generator can be implantable, or the noise generator can be positioned external to the patient.

In an additional embodiment, the broad spectrum of electrical noise signals can include Gaussian noise, white noise, pink noise, Brownian noise, grey noise, or a combination thereof.

In another embodiment, only tuned electrical noise signals can be delivered to the target neural tissue, non-neural tissue, or a combination thereof.

A method for providing therapy to a patient may include delivering a broad spectrum of electrical noise signals to target neural tissue, non-neural tissue, or a combination thereof in the patient via an electrode, wherein the broad spectrum of electrical noise signals are tunable; and using feedback from the patient to tune the broad spectrum of electrical noise signals to optimize the therapy provided to the patient.

In one embodiment of the method, the broad spectrum of electrical noise signals can be tuned by adjusting energy contained within a frequency band.

In another embodiment, the broad spectrum of electrical noise signals can be tuned by adjusting a phase component of the broad spectrum of electrical noise signals.

In yet another embodiment, the therapy provided to the patient can treat pain.

In one more embodiment, the therapy provided to the patient can treat an autonomic disorder.

In an additional embodiment, the therapy provided to the patient can treat a sensory disorder.

In another embodiment, the therapy provided to the patient can treat a motor disorder.

In one particular embodiment, the therapy provided to the patient can elicit plastic changes in neural tissue, non-neural tissue, or a combination thereof to mitigate or abolish a pathophysiologic disease or syndrome.

In still another embodiment, the target neural tissue, non-neural tissue, or a combination thereof can be tissue located within or adjacent the patient's brain, the patient's spinal cord, a dorsal root ganglion, a sympathetic chain ganglion, or a peripheral nerve.

In yet another embodiment, the electrode can be percutaneous, transcutaneous, or implantable.

In one more embodiment, the electrode can be coupled to a noise generator and a controller. Further, the noise generator can be implantable or the noise generator can be positioned external to the patient, while the controller can be configured to tune the broad spectrum of electrical noise signals.

In an additional embodiment, the broad spectrum of electrical noise signals can include Gaussian noise, white noise, pink noise, Brownian noise, grey noise, or a combination thereof.

In another embodiment, only tuned electrical noise signals are delivered to the target neural tissue, non-neural tissue, or a combination thereof.

These and other features and advantages of the invention will become more apparent to one skilled in the art from the following description and claims when read in light of the accompanying drawings.

### BRIEF DESCRIPTION OF THE DRAWINGS

A full and enabling disclosure of the present invention, including the best mode thereof, directed to one of ordinary skill in the art, is set forth more particularly in the remainder of the specification, which makes reference to the appended figures in which:
FIG. 1 illustrates one system for delivering a broad spectrum of electrical noise signals to target neural tissue, non-neural tissue, or a combination thereof in a patient, where the electrical noise signals are tunable, and where the target tissue is located within or adjacent to the patient's brain.
FIG. 2 illustrates one system for delivering a broad spectrum of electrical noise signals to target neural tissue, non-neural tissue, or a combination thereof in a patient, where the electrical noise signals are tunable, and where the target tissue is located within or adjacent the spinal cord;
FIG. 3 is a zoomed-in view of the spinal cord and illustrates one option for electrode placement according to the system of FIG. 2, where the target tissue is located within or adjacent a dorsal region of the spinal cord, such as the dorsal columns;
FIG. 4 is a zoomed-in view of the spinal cord and illustrates another option for electrode placement according to the system of FIG. 2, where the target tissue is located within or adjacent to the dorsolateral region of the spinal cord, such as the dorsolateral funiculus.
FIG. 5 is a zoomed-in view of the spinal cord and illustrates one more option for electrode placement according to the system of FIG. 2, where the target tissue is located within or adjacent the lateral region of the spinal cord, such as the spinothalamic tract;
FIG. 6 is a zoomed-in view of the spinal cord and illustrates yet another option for electrode placement according to the system of FIG. 2, where the target tissue is located with or adjacent the ventral region of the spinal cord, such as the ventral horn;
FIG. 7 is a zoomed-in view of the spinal cord and illustrates one option for electrode placement according to the system of FIG. 2, where the target tissue is located within or adjacent a dorsal root ganglion;
FIG. 8 illustrates one system for delivering a broad spectrum of electrical noise signals to target neural tissue, non-neural tissue, or a combination thereof in a patient, where the electrical noise signals are tunable, and where the target tissue is located within or adjacent a sympathetic chain ganglion;
FIG. 9 is a zoomed-in view of the sympathetic chain and illustrates one option for electrode placement according to the system of FIG. 8;
FIG. 10 illustrates one system for delivering a broad spectrum of electrical noise signals to target neural tissue, non-neural tissue, or a combination thereof in a patient, where the electrical noise signals are tunable, and where the target tissue is located within or adjacent a peripheral nerve;
FIG. 11A is a graph of an original amplitude spectrum for a broad spectrum of electrical noise signals; and
FIG. 11B is a graph of an amplitude spectrum for a broad spectrum of electrical noise signals that has been tuned based on patient feedback.

Repeat use of reference characters in the present specification and drawings is intended to represent same or analogous features or elements of the invention.

### DEFINITIONS

As used herein, the term "electrical noise signal" refers to a random electrical signal that can be applied to target neural tissue, non-neural tissue, or a combination thereof. The electrical noise signal can include Gaussian noise, white noise, pink noise, red (Brownian) noise, grey noise, or a multifaceted noise containing a combination of these and any other suitable noise signals as discussed in more detail below. The electrical noise signal can be band limited to an upper cutoff frequency and a lower cutoff frequency that are broader than the natural resonant frequencies of the modulated neuronal circuitry and electrical stimulation paradigms practiced today. Further, the electrical noise signal is distinguished from a traditional electrical stimulation signal in that it is a random signal that varies in an unpredictable manner over time, or is aperiodic. Traditional electrical stimulation signals are periodic waveforms that are predictable. A periodic waveform is not utilized in the electrical noise signal of the present invention. In other words, the electrical noise signal is aperiodic, and unlike periodic signals used for stimulation today, the tuned electrical noise signal enables independent adjustment of the energy content within all frequencies of its frequency spectrum.

### DETAILED DESCRIPTION OF THE INVENTION

Reference will now be made in detail to various embodiments of the invention, one or more examples of which are set forth below. Each example is provided by way of explanation of the invention, not limitation of the invention. In fact, it will be apparent to those skilled in the art that various modifications and variations may be made in the present invention without departing from the scope or spirit of the invention. For instance, features illustrated or described as part of one embodiment, may be used on another embodiment to yield a still further embodiment. Thus, it is intended that the present invention covers such modifications and variations as come within the scope of the appended claims and their equivalents.

Generally speaking, a method and system for providing therapy to a patient via delivery of a broad spectrum of tunable electrical noise signals are described. The method includes delivering a broad spectrum of tunable electrical noise signals to target neural tissue, non-neural tissue, or a combination thereof in the patient via an electrode, and using feedback from the patient to tune the broad spectrum of electrical noise signals to optimize the therapy provided to the patient. The system includes an electrode, a noise generator coupled to the electrode, and a controller. The controller instructs the noise generator to deliver a broad spectrum of tunable electrical noise signals to target neural tissue, non-neural tissue, or a combination thereof via the electrode, and the controller is configured to tune the broad spectrum of electrical noise signals to optimize the therapy provided to the patient based on feedback received from the patient.

For example, the method and system can include applying a broad spectrum of tunable electrical noise signals to target neural tissue, non-neural tissue, or a combination thereof located within or adjacent the patient's brain or spinal cord, a dorsal root ganglion, a sympathetic chain ganglion, or a peripheral nerve, as described in more detail below, where the delivery of the broad spectrum of tunable electrical noise signals can be tuned or adjusted based on patient feedback to treat a specific condition, illness, disease state, symptom, etc.

Specifically, the therapy provided to the patient through the broad spectrum of tunable electrical noise signals can treat pain (e.g., chronic pain), an autonomic disorder (e.g., hypertension, hypotension, complex regional pain syndrome (CRPS), Raynaud's syndrome, etc.), a sensory disorder (e.g., tinnitus, hearing loss, vertigo, etc.), a motor disorder (e.g., Huntington's disease, Parkinson's disease, Multiple Sclerosis, spinal muscular atrophy (SMA), dystonia, essential tremor, etc.), or a combination thereof. Further, the therapy provided to the patient can elicit plastic changes in neural tissue, non-neural tissue, or a combination thereof to mitigate or abolish a pathophysiologic disease or syndrome. Plastic changes are changes to the neural tissue, non-neural tissue, or a combination thereof in response to physiological demands. Such plastic changes can include morphological and functional changes.

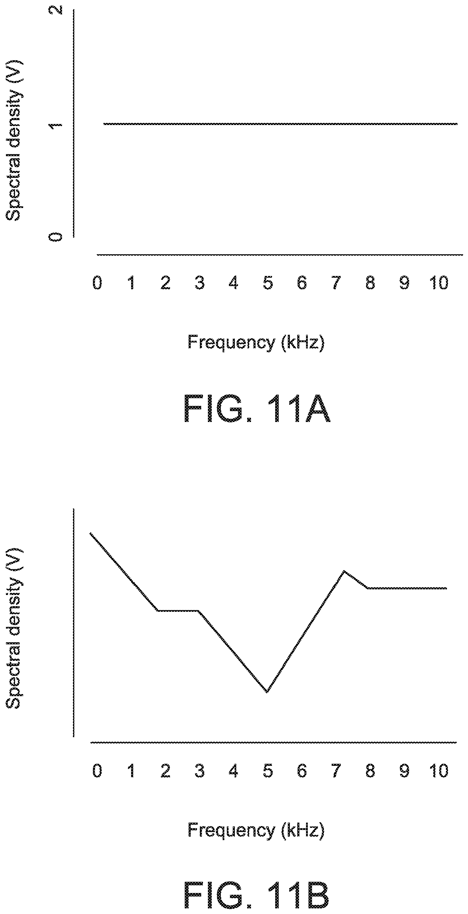

In one particular embodiment, the broad spectrum of electrical noise signals can be tuned based on patient feedback by adjusting energy contained within a frequency band, while in another embodiment, the broad spectrum of electrical noise signals can be tuned based on patient feedback by adjusting a phase component of the broad spectrum. For example, one or more electrodes can be implanted, inserted percutaneously, or positioned transcutaneously such that the electrodes are nearby the target neural tissue, non-neural tissue and combination thereof as necessary to treat their disease or syndrome. A noise generator can then be instructed to deliver a broad spectrum of electrical noise signals through the one or more electrodes. The patient and/or caregiver can then program the optimal stimulation waveform by operating a controller. The controller can tune the waveform associated with the broad spectrum of electrical noise signals being delivered to the patient by adjusting energy levels within a particular frequency band, and for all frequency bands delivered, to best treat the patient. For example, FIG. 11A shows the spectral density of the original broad spectrum of electrical noise signals, while FIG. 11B shows the broad spectrum of electrical noise signals after it has been tuned to best treat the particular patient based on feedback received from the patient.

Regardless of the specific manner in which the broad spectrum of electrical noise signals are tuned based on patient feedback, the tunability feature contemplated by the method and by the system of the present invention allows for the therapy provided to the patient to be tuned, altered, adjusted, etc. based on the specific disease state being treated, the physiological characteristics of the patient, and/or the current activity level of the patient, where each of these variables can affect how the originally applied broad spectrum of electrical noise signals improves the patient's symptoms.

Whether the broad spectrum of tunable electrical noise signals is being applied to target neural tissue, non-neural tissue, or a combination thereof located within or adjacent the patient's brain or spinal cord, a dorsal root ganglion, a sympathetic chain ganglion, or a peripheral nerve, the present inventor has found that the specific parameters of the broad spectrum of tunable electrical noise signals and the location of the electrodes through which the broad spectrum of tunable electrical noise signals is delivered can be selectively controlled to provide improved symptom relief and therapy to the patient for the treatment of pain, autonomic disorders, sensory disorders, motor disorders, etc. The specific system and parameters are discussed in more detail below.

### System for Delivering the Broad Spectrum of Tunable Electrical Noise Signals

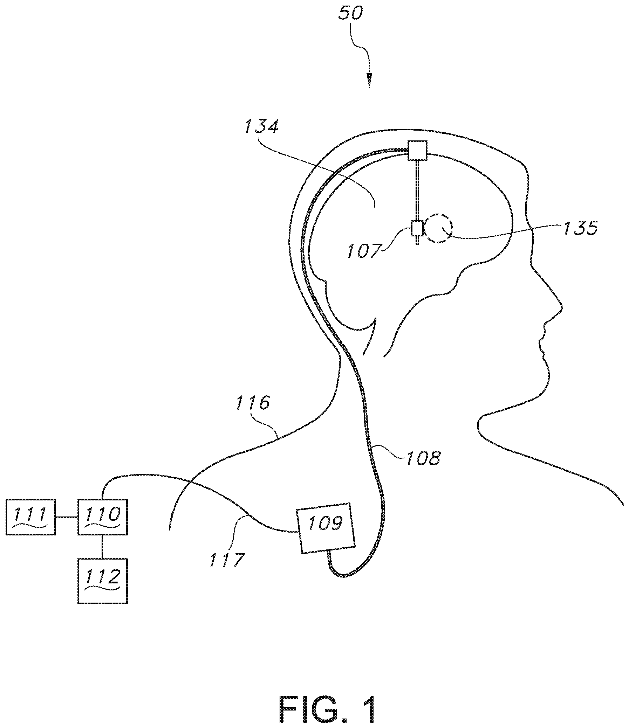

Referring now to FIG. 1 of the drawings, there is illustrated a system 50 for delivering a broad spectrum of tunable electrical noise signals to provide therapy to a patient 116, where the target neural tissue, non-neural tissue, or a combination thereof 135 is located within or adjacent tissue within the patient's brain 134. In general, the system 50 in FIG. 1 can include one or more electrodes 107 (shown diagrammatically in FIG. 1 and not in any specific detail) that are connected by an electrical lead 108 to a noise generator 109. An additional lead 117 can be used to couple the noise generator 109 to the rest of the system 50, which can include a user interface 112 and a controller 110, where it is to be understood that as an alternative to the use of the lead 117, the noise generator 109 can be wirelessly connected to the rest of the system 50. The system can also include a power system 111 and an optional patient monitor system. Further, it should be understood that while the system 50 of FIG. 1 illustrates a configuration where a broad spectrum of tunable electrical noise signals can be delivered to target neural tissue, non-neural tissue, or a combination 135 thereof utilizing an electrode 107 coupled to an implantable noise generator 109 via a lead 108, the electrode 107 can alternatively be coupled to an external noise generator (not shown) via a wireless antenna system (not shown). In addition, more than one electrode 107 can be used. Regardless of the exact type (e.g., percutaneous, transcutaneous, implantable, etc.) or configuration (e.g., monopolar, bipolar, multipolar, etc.) of the electrode(s) 107, the electrode(s) 107 can be in the form of an electrode assembly that can deliver a broad spectrum of tunable electrical noise signals to a patient to provide therapy to the patient and/or improve one or more of the patient's symptoms. Specific diseases or conditions that can be treated based on stimulation of the brain, include: Parkinson's disease, essential tremor, depression, obsessive compulsive disorder, Tourette's syndrome, epilepsy, schizophrenia, narcolepsy, seizures, Alzheimer's disease, tinnitus, Meniere's disease, and chronic pain.

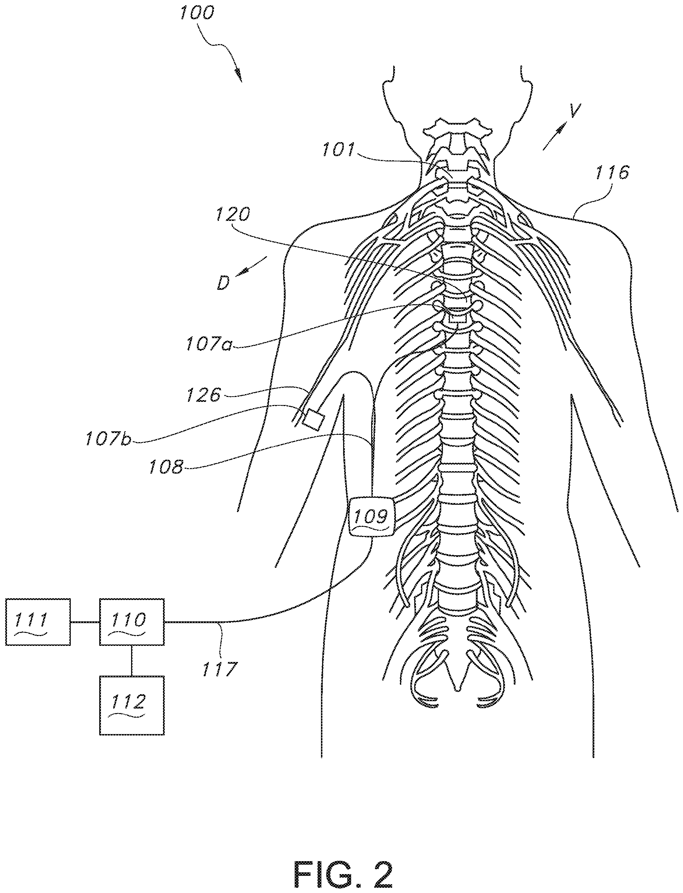

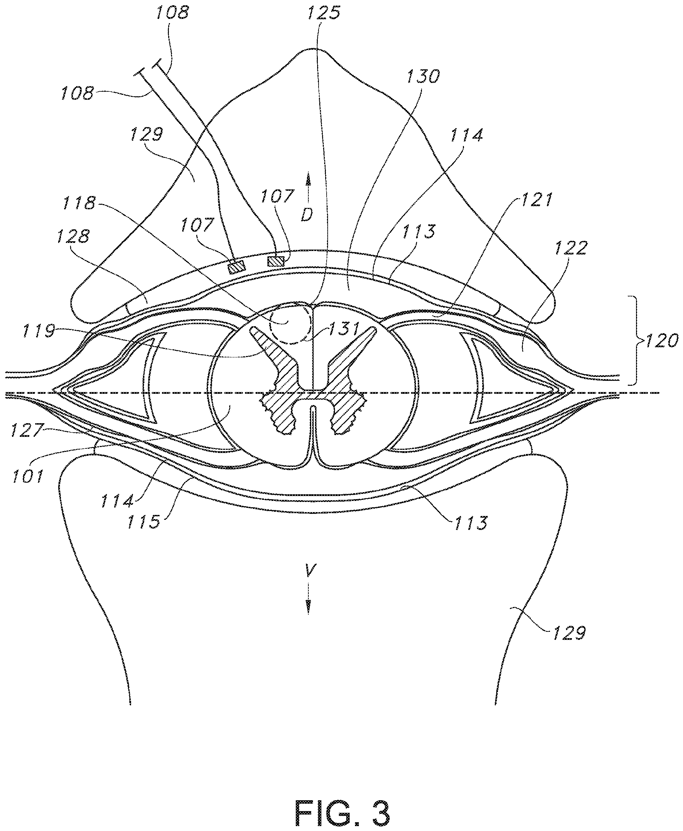

Referring next to FIG. 2 of the drawings, there is illustrated a system 100 for delivering a broad spectrum of tunable electrical noise signals to provide therapy to a patient, where the target neural tissue, non-neural tissue, or a combination thereof is located within or adjacent the spinal cord 101 of a patient 116. As shown in FIG. 2, the system 100 can include multiple devices to control and deliver a broad spectrum of tunable electrical noise signals to one or more areas of target neural tissue, non-neural tissue, or a combination thereof located within or adjacent the spinal cord 101 to provide therapy to a patient 116. In general, the system 100 in FIG. 2 can include one or more electrodes 107a and/or 107b (shown diagrammatically in FIG. 2 and not in any specific detail) that are connected by an electrical lead 108 to a noise generator 109. An additional lead 117 can be used to couple the noise generator 109 to the rest of the system 100, which can include a user interface 112 and a controller 110, where it is to be understood that as an alternative to the use of the lead 117, the noise generator 109 can be wirelessly connected to the rest of the system 50. The system can also include an isolated power system 111 and an optional patient monitor system. Further, it should be understood that while the system 100 of FIG. 2 illustrates a configuration where a broad spectrum of tunable electrical noise signals can be delivered to target neural tissue, non-neural tissue, or a combination thereof utilizing electrodes 107a and/or 107b coupled to an implantable noise generator 109 via a lead 108, the electrodes 107a and/or 107b can alternatively be coupled to an external noise generator (not shown) via a wireless antenna system (not shown). Regardless, the electrodes 107a and/or 107b can be in the form of an electrode assembly that can deliver a broad spectrum of tunable electrical noise signals to a patient to provide therapy to the patient and/or improve one or more of the patient's symptoms based on the specific location of the electrodes, as discussed in more detail in FIGs. 3-7 below.

Turning now to FIG. 3, the placement of the electrode or electrodes 107 in order to deliver a broad spectrum of tunable electrical noise signals to an area within or adjacent target neural tissue, non-neural tissue, or a combination thereof 131 located adjacent a dorsal region 120 of the spinal cord 101, and in particular a dorsal column 118, is discussed in more detail, where the dorsal D and ventral V directions of the spinal cord 101 are labeled for reference purposes. For instance, one or more electrodes 107 can be positioned within a portion of the epidural space 128 of the patient 116 adjacent a dorsal region 120 of the spinal cord 101, where the dorsal region 120 of the spinal cord 101 can be identified via locating the posterior median sulcus 125. As shown, the epidural space 128 is positioned between the bone 129 (vertebrae) and the dura mater 115. Thus, a broad spectrum of tunable electrical noise signals transmitted by the electrode 107 must be configured to pass through the dura mater 115, subdural cavity 113, arachnoid mater 114, subarachnoid cavity 130, and pia mater 127 to reach the target neural tissue, non-neural tissue, or a combination thereof 131 and deliver the desired broad spectrum of tunable electrical noise signals therein. The present inventor has found that by placing the electrode or electrodes 107 in the epidural space 128 adjacent a dorsal region 120 of the spinal cord 101, a broad spectrum of tunable electrical noise signals can be delivered to target neural tissue, non-neural tissue, or a combination thereof 131 located within or adjacent a dorsal column 118 to provide therapy to the patient. It is also to be understood that the electrode or electrodes 107 can be positioned in any suitable location in the dorsal region 120 of the spinal cord 101 in order to deliver a broad spectrum of tunable electrical noise signals to an area within or adjacent other target neural tissue, non-neural tissue, or a combination thereof, such as tissue located adjacent a dorsal horn 119 or a dorsal root 121. Specific diseases or conditions that can be treated based on stimulation of the dorsal region of the spinal cord, and in particular, the dorsal columns include: acute pain, failed back surgery syndrome, complex regional pain syndrome, peripheral vascular disease and chronic limb ischemia, angina pain, diabetic pain, abdominal/ visceral pain syndrome, brachial plexitis, phantom limb pain, intractable pain secondary to spinal cord injury, mediastinal pain, Raynaud's syndrome, cervical neuritis, post herpetic neuralgia, vertigo, tinnitus, hearing loss and inflammatory pain such as arthritis, irritable bowel pain, osteoarthritis pain and fibromyalgia.

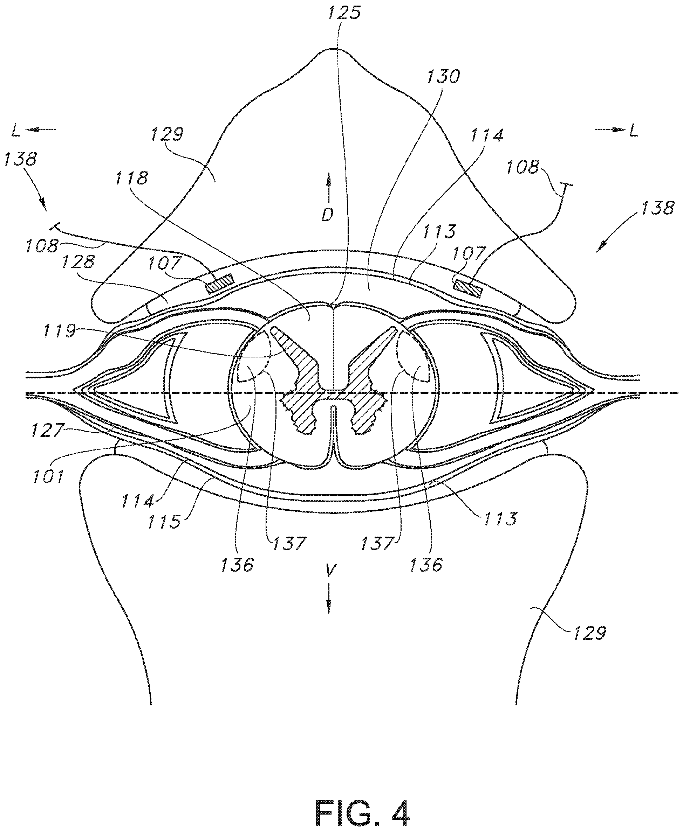

Referring now to FIG. 4, the placement of the electrode or electrodes 107 in order to deliver a broad spectrum of tunable electrical noise signals to an area within or adjacent target neural tissue, non-neural tissue, or a combination thereof 137 located in a dorsolateral region 138 of the spinal cord 101 is discussed in more detail, where the dorsal D, ventral V, and lateral L directions are labeled for reference purposes. For instance, one or more electrodes 107 can be positioned adjacent a dorsolateral region 138 of the spinal cord 101. The present inventor has found that by placing the electrode or electrodes 107 adjacent a dorsolateral region 138 of the spinal cord 101, a broad spectrum of tunable electrical noise signals can be delivered to target neural tissue, non-neural tissue, or a combination thereof 137 located within or adjacent a dorsolateral region 138 of the spinal cord 101 to provide therapy to the patient. Specifically, nerve fiber activity in the right or left dorsolateral funiculus 136 or a combination thereof can be altered via a broad spectrum of tunable electrical noise signals in order to treat or alleviate symptoms associated various conditions. Specific diseases or conditions that can be treated based on stimulation of the dorsolateral region of the spinal cord, and in particular, the dorsolateral funiculus include: acute pain, failed back surgery syndrome, complex regional pain syndrome, peripheral vascular disease and chronic limb ischemia, angina pain, diabetic pain, abdominal/ visceral pain syndrome, brachial plexitis, phantom limb pain, intractable pain secondary to spinal cord injury, mediastinal pain, Raynaud's syndrome, cervical neuritis, post herpetic neuralgia, vertigo, tinnitus, hearing loss and inflammatory pain such as arthritis, irritable bowel pain, osteoarthritis pain and fibromyalgia.

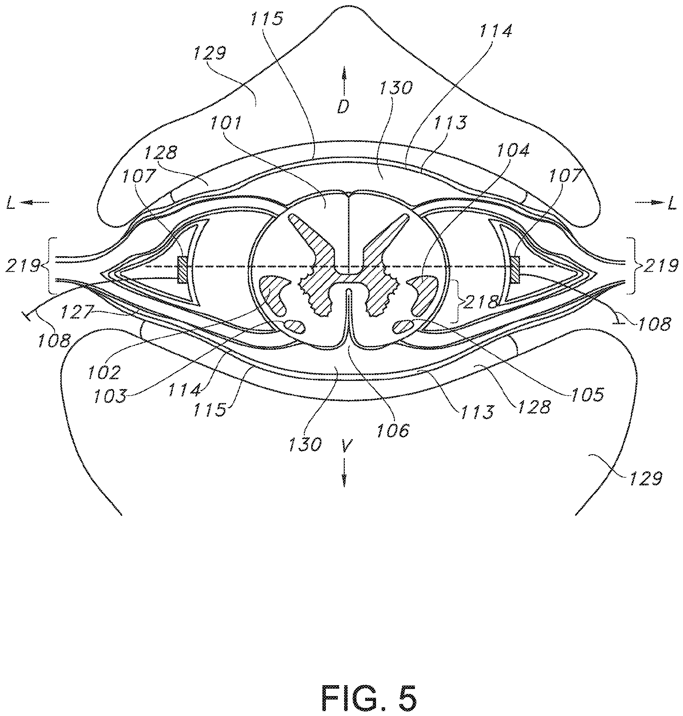

Turning now to FIG. 5, the placement of the electrode or electrodes 107 in order to deliver a broad spectrum of tunable electrical noise signals to an area within or adjacent target neural tissue, non-neural tissue, or a combination thereof 218 located in a lateral region 219 of the spinal cord 101 is discussed in more detail, where the dorsal D, ventral V, and lateral L directions are labeled for reference purposes. For instance, one or more electrodes 107 can be positioned adjacent a lateral region 219 of the spinal cord 101. The present inventor has found that by placing the electrode or electrodes 107 adjacent lateral region 219 of the spinal cord 101, a broad spectrum of tunable electrical noise signals can be delivered to target neural tissue, non-neural tissue, or a combination thereof 218 located within or adjacent a lateral region 219 of the spinal cord 101 to provide therapy to the patient. Specifically, nerve fiber activity in the right lateral spinothalamic tract 102, the left lateral spinothalamic tract 104, or a combination thereof can be altered via a broad spectrum of tunable electrical noise signals in order to treat or alleviate symptoms associated various conditions. Moreover, it is to be understood that nerve fiber activity in the right anterior spinothalamic tract 103, the left anterior spinothalamic tract 105, or a combination thereof can also be altered via a broad spectrum of tunable electrical noise signals based on the specific positioning of the one or more electrodes 107. Specific diseases or conditions that can be treated based on stimulation of the lateral region of the spinal cord, and in particular, the lateral spinothalamic tract include: acute pain, failed back surgery syndrome, complex regional pain syndrome, peripheral vascular disease and chronic limb ischemia, angina pain, diabetic pain, abdominal/ visceral pain syndrome, brachial plexitis, phantom limb pain, intractable pain secondary to spinal cord injury, mediastinal pain, Raynaud's syndrome, cervical neuritis, post herpetic neuralgia, vertigo, tinnitus, hearing loss and inflammatory pain such as arthritis, irritable bowel pain, osteoarthritis pain and fibromyalgia.

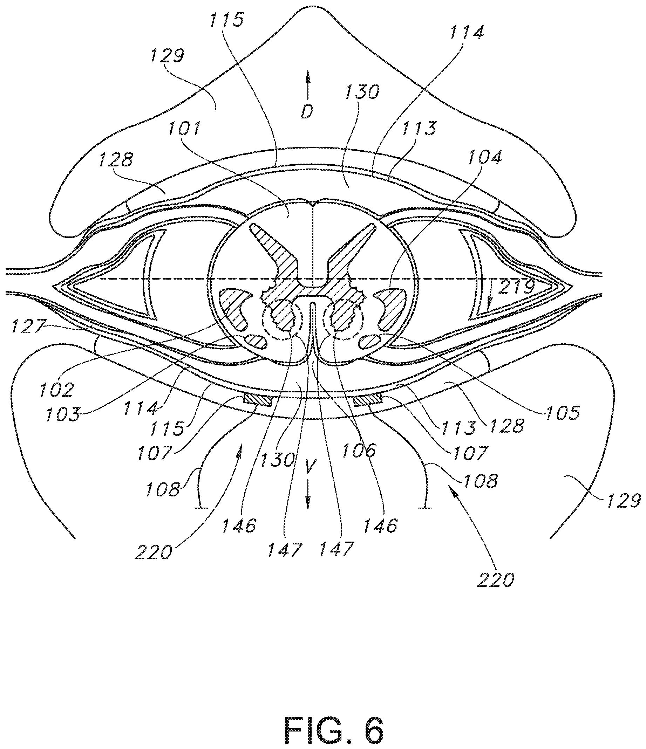

Turning now to FIG. 6, the placement of the electrode or electrodes 107 in order to deliver a broad spectrum of tunable electrical noise signals to an area within or adjacent target neural tissue, non-neural tissue, or a combination thereof 147 located in a ventral region 220 of the spinal cord 101 is discussed in more detail, where the dorsal D and ventral V directions are labeled for reference purposes. For instance, one or more electrodes 107 can be positioned within a portion of the epidural space 128 of the patient 116 adjacent a ventral region 220 of the spinal cord 101, where the ventral region 220 of the spinal cord 101 can be identified via locating the anterior median fissure 106. As shown, the epidural space 128 is positioned between the bone 129 (vertebrae) and the dura mater 115. Thus, a broad spectrum of tunable electrical noise signals transmitted by the electrode 107 must be configured to pass through the dura mater 115, subdural cavity 113, arachnoid mater 114, subarachnoid cavity 130, and pia mater 127 to reach the target neural tissue, non-neural tissue, or a combination thereof 147 and deliver the desired broad spectrum of tunable electrical noise signals therein. The present inventor has found that by placing the electrode or electrodes 107 in the epidural space 128 adjacent a ventral region 220 of the spinal cord 101, a broad spectrum of tunable electrical noise signals can be delivered to target neural tissue, non-neural tissue, or a combination thereof 147 located in a ventral region 220 of the spinal cord 101 to provide therapy to the patient. Specifically, in one particular embodiment, nerve fiber activity in the right or left ventral horn 146 or a combination thereof can be altered via a broad spectrum of tunable electrical noise signals in order to treat or alleviate symptoms associated various conditions. Specific diseases or conditions that can be treated based on stimulation of the ventral region of the spinal cord include: motoneuron disease (amyotrophic lateral sclerosis; progressive muscular atrophy; progressive bulbar palsy; primary lateral sclerosis; hereditary spastic paraplegia), spinal muscular atrophy (infantile and juvenile spinal muscular atrophy; focal amyotrophy), and multiple sclerosis.

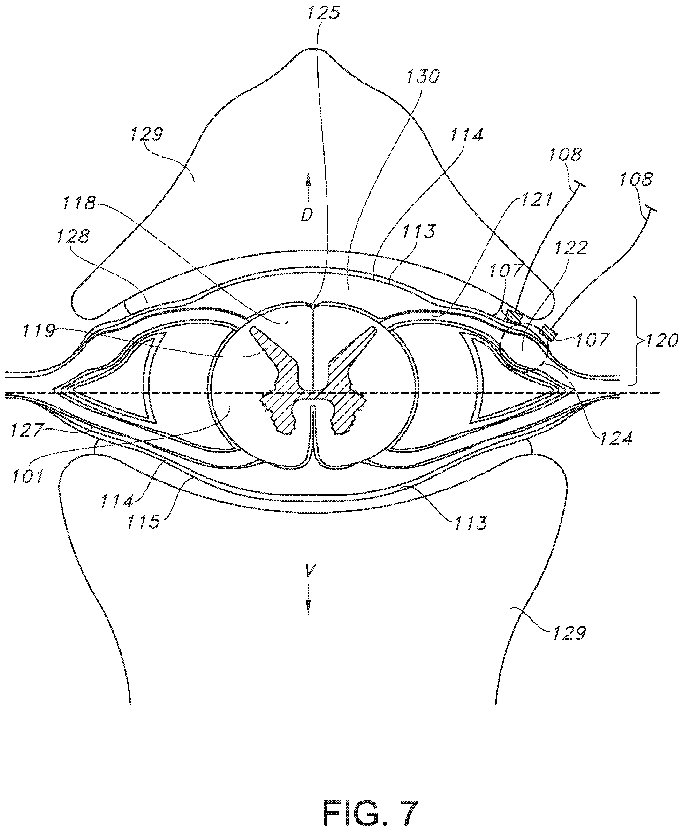

Turning now to FIG. 7, the placement of the electrode or electrodes 107 in order to deliver a broad spectrum of tunable electrical noise signals to an area within or adjacent target neural tissue, non-neural tissue, or a combination thereof 124 located adjacent or near a dorsal region 120 of the spinal cord 101, and in particular a dorsal root ganglion 122, is discussed in more detail, where the dorsal D and ventral V directions of the spinal cord 101 are labeled for reference purposes. For instance, one or more electrodes 107 can be positioned within a portion of the epidural space 128 of the patient 116 adjacent a dorsal (or posterior) portion 120 of the spinal cord 101, where the dorsal (or posterior) portion 120 of the spinal cord 101 can be identified via locating the posterior median sulcus 125. As shown, the epidural space 128 is positioned between the bone 129 (vertebrae) and the dura mater 115. Thus, a broad spectrum of tunable electrical noise signals transmitted by the electrode 107 must be configured to pass through the dura mater 115, subdural cavity 113, arachnoid mater 114, subarachnoid cavity 130, and pia mater 127 to reach the target neural tissue, non-neural tissue, or a combination thereof 124 and deliver the desired broad spectrum of tunable electrical noise signals therein. The present inventor has found that by placing the electrode or electrodes 107 in the epidural space 128 adjacent a dorsal D (or posterior) portion 120 of the spinal cord 101, a broad spectrum of tunable electrical noise signals can be delivered to target neural tissue, non-neural tissue, or a combination thereof 124 located within or adjacent a dorsal root ganglion 122 to provide therapy to the patient. Specific diseases or conditions that can be treated based on stimulation of the dorsal root ganglion include: acute pain, failed back surgery syndrome, complex regional pain syndrome, peripheral vascular disease and chronic limb ischemia, angina pain, diabetic pain, abdominal/ visceral pain syndrome, brachial plexitis, phantom limb pain, intractable pain secondary to spinal cord injury, mediastinal pain, Raynaud's syndrome, cervical neuritis, post herpetic neuralgia, vertigo, tinnitus, hearing loss and inflammatory pain such as arthritis, irritable bowel pain, osteoarthritis pain and fibromyalgia.

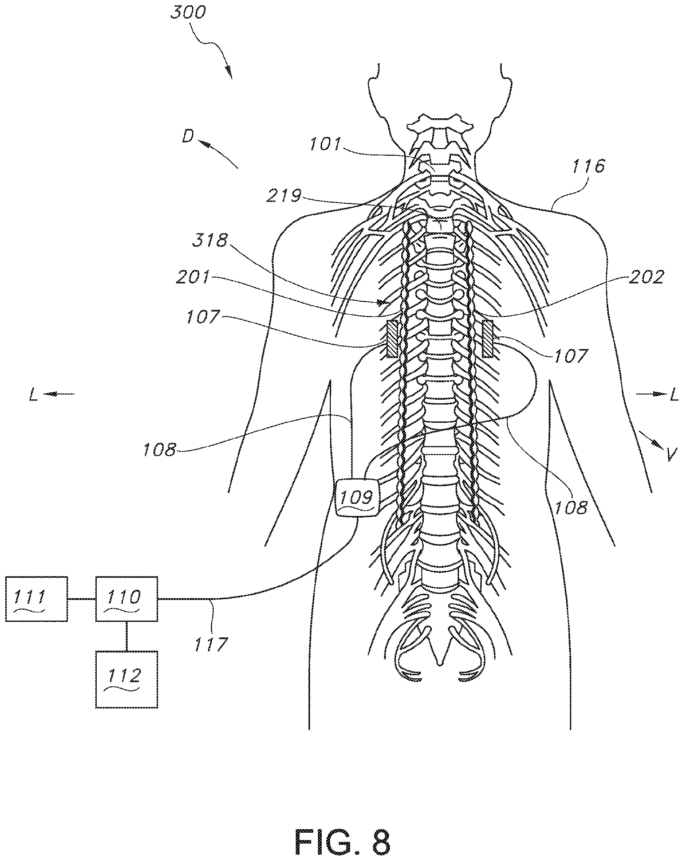

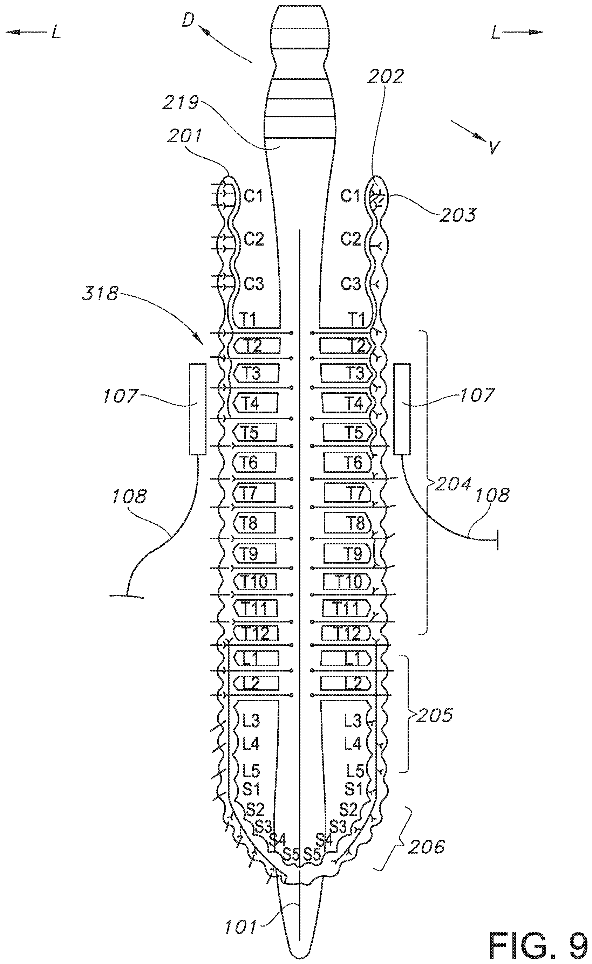

Referring now to FIG. 8, there is illustrated a system 300 for delivering a broad spectrum of tunable electrical noise signals to provide therapy to a patient 116, where the target neural tissue, non-neural tissue, or a combination thereof 318 located adjacent a ventral or anterior region 219 of a spinal cord 101 of the patient 116. In particular, the target neural tissue, non-neural tissue, or a combination thereof 318 can be a sympathetic chain ganglion located in the right sympathetic chain 201, the left sympathetic chain 202, or a combination thereof. As shown in FIG. 9, the system 300 can include multiple devices to control and deliver a broad spectrum of tunable electrical noise signals to an area within or adjacent target neural tissue, non-neural tissue, or a combination thereof 318 located adjacent a ventral V (or anterior) region 219 of the spinal cord 101 to provide therapy to the patient 116. In general, the system 300 in FIG. 8, can include one or more electrodes 107 (shown diagrammatically in FIG. 8 and not in any specific detail) that are connected by an electrical lead 108 to a noise generator 109. An additional lead 117 can be used to couple the noise generator 109 to the rest of the system 300, which can include a user interface 112, and a controller 110, where it is to be understood that as an alternative to the use of the lead 117, the noise generator 109 can be wirelessly connected to the rest of the system 50. The system may also include an isolated power system 111 and an optional patient monitor system. Further, it should be understood that while the system 300 of FIG. 8 illustrates a configuration where a broad spectrum of tunable electrical noise signals can be delivered to target neural tissue, non-neural tissue, or a combination thereof utilizing an electrode or electrodes 107 coupled to an implantable noise generator 109 via a lead 108, the electrode or electrodes 107 can alternatively be coupled to an external noise generator (not shown) via a wireless antenna system (not shown). Regardless, the electrode or electrodes 107 can be in the form of an electrode assembly that can that can deliver a broad spectrum of tunable electrical noise signals to a patient to provide therapy to the patient and/or improve one or more of the patient's symptoms.

Turning now to FIG. 9, the placement of the electrode or electrodes 107 is discussed in more detail. For instance, one or more electrodes 107 can be positioned adjacent a region of the right sympathetic chain 201 or the left sympathetic chain 202 of the patient 116, where the sympathetic chains 201 and 202 are located ventral and lateral to a ventral (or anterior) region 219 of the spinal cord 101. By placing the electrode or electrodes 107 adjacent target neural tissue, non-neural tissue, or a combination thereof 318 located lateral and ventral to a ventral (or anterior) region 219 of the spinal cord 101, a broad spectrum of tunable electrical noise signals can be delivered to the target neural tissue, non-neural tissue, or a combination thereof 318 (e.g., a ganglion or ganglia of the right sympathetic chain 201 or the left sympathetic chain 202) to provide therapy to the patient.

For instance, a broad spectrum of tunable electrical noise signals can be delivered to a ganglion or ganglia associated with the cervical portion 203, the thoracic portion 204, the lumbar portion 205, or the sacral portion 206 of the right sympathetic chain 201 or the left sympathetic chain 202, or any combination thereof to provide therapy to the targeted area or areas. In one particular embodiment, an electrode 107 can be placed adjacent the cervical region 203 of the sympathetic chain to affect nerve fiber activity associated with levels C1-C3, which can affect nerve fiber activity associated with the eyes, the lachrymal glands, the salivary glands, and the sweat glands, hair follicles, and blood vessels of the head, neck, and arms. In another embodiment, an electrode 107 can be placed adjacent levels T1-T4 of the thoracic region 204, which can affect nerve fiber activity associated with the heart and lungs. In an additional embodiment, an electrode 107 can be placed adjacent levels T5-T9 of the thoracic region 204, which can affect nerve fiber activity associated with the stomach, duodenum, pancreas, liver, kidneys, and adrenal medulla. In yet another embodiment, an electrode 107 can be placed adjacent levels T10-T11 of the thoracic region 204, which can affect nerve fiber activity associated with the stomach and duodenum. In one more embodiment, an electrode 107 can be placed adjacent level T12 of the thoracic region 204 and levels L1-L3 of the lumbar region 205, which can affect nerve fiber activity in the colon, rectum, bladder, and external genitalia. In still another embodiment, an electrode 107 can be placed adjacent levels L4-L5 of the lumbar region 205 and levels S1-S3 of the sacral region 206, which can affect nerve fiber activity associated with the sweat glands, hair follicles, and blood vessels of the lower limbs. In another embodiment, an electrode 107 can be placed adjacent levels S4-S5 of the sacral region 206, which can affect nerve fiber activity associated with the sweat glands, hair follicles, and blood vessels of the perineum. Specific diseases or conditions that can be treated based on stimulation of a sympathetic nervous system include: complex regional pain syndrome, peripheral vascular disease and chronic limb ischemia, angina pain, diabetic pain, abdominal/ visceral pain syndrome, phantom limb pain, Raynaud's syndrome, hypertension, hypotension, headache and migraine, and inflammatory pain such as arthritis, irritable bowel pain, osteoarthritis pain and fibromyalgia.

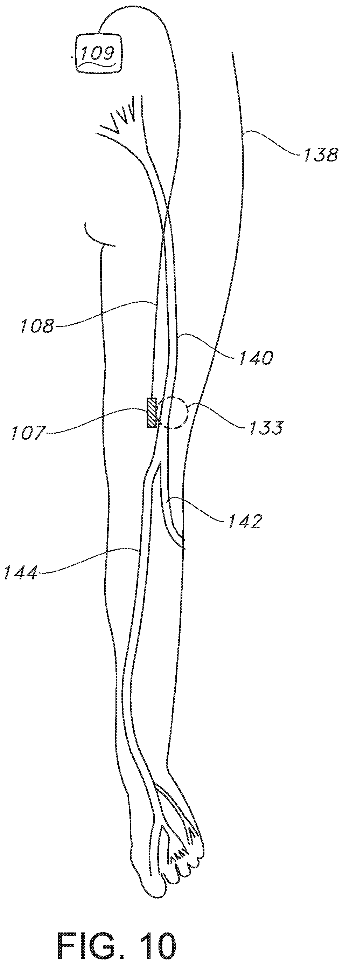

Turning now to FIG. 10, the placement of the electrode or electrodes 107 in order to deliver a broad spectrum of tunable electrical noise signals to an area within or adjacent target neural tissue, non-neural tissue, or a combination thereof 133 located adjacent or near a peripheral nerve is discussed in more detail. For instance, one or more electrodes 107 can be positioned near or adjacent a peripheral nerve at any location along its length, where the peripheral nerve can run, for instance, down the length of the leg 138 of the patient 116. In the particular embodiment of FIG. 10, the target tissue 133 is located adjacent the sciatic nerve 140, although it is to be understood that neural tissue, non-neural tissue, or a combination thereof can be located adjacent any peripheral nerve in the leg (e.g., the common peroneal nerve 142, the tibial nerve 144, etc.), or any other location in the body. The present inventor has found that by placing the electrode or electrodes 107 adjacent or near a peripheral nerve, a broad spectrum of tunable electrical noise signals can be delivered to the target neural tissue, non-neural tissue, or a combination thereof 133 to provide therapy to the patient. Specific diseases or conditions that can be treated based on stimulation of a peripheral nerve include: acute pain, failed back surgery syndrome, complex regional pain syndrome, peripheral vascular disease and chronic limb ischemia, angina pain, diabetic pain, abdominal/ visceral pain syndrome, brachial plexitis, phantom limb pain, intractable pain secondary to spinal cord injury, mediastinal pain, Raynaud's syndrome, headache and migraine, cervical neuritis, post-herpetic neuralgia, vertigo, tinnitus, hearing loss and inflammatory pain such as arthritis, irritable bowel pain, osteoarthritis pain and fibromyalgia.

The various components of the systems 50, 100, and 300 described in FIGs. 1-10 are discussed in more detail below.

Electrodes. It is to be understood that one or more electrodes 107 can be used to deliver the broad spectrum of tunable electrical noise signals to the target neural tissue, non-neural tissue, or a combination thereof. Further, it is to be understood that the one or more electrodes 107 can be implantable. In other embodiments, the electrodes can be percutaneous or transcutaneous. In addition, it is to be understood that the one or more electrodes can have a monopolar, bipolar, or multipolar configuration. For example, an electrode used in a bipolar or multipolar fashion has at least one cathode and one anode in the vicinity of the target neural tissue, non-neural tissue, or a combination thereof, while a monopolar electrode can have a cathode located nearby the target neural tissue, non-neural tissue, or a combination thereof, and a return electrode positioned some distance away. Further, the electrode shape and size, and inter-electrode spacing can be specific to contouring the electrical field surrounding the target neural tissue, non-neural tissue, or a combination thereof, to enable specific therapy to be provided to the target neural tissue, non-neural tissue, or a combination thereof.

Noise Generator. As shown in the figures, the electrode or electrodes 107 may be connected to an implantable noise generator 109 through an electrical lead 108. Alternatively, the noise generator 109 can be external and can be wirelessly connected to the electrode or electrodes 107. In one particular embodiment, the noise generator 109 can be configured to deliver a broad spectrum of tunable electrical noise signals to provide therapy to a patient that can be customized based on patient feedback, where the therapy provided can depend on the specific disease state being treated, the physiological characteristics of the patient, and/or the current activity level of the patient.

User interface. The systems 100, 200, and/or 300 may utilize a user interface 112. The user interface 112 can be in the form of a computer that interacts with the controller 110 and is powered by a power system 111, each described herein.

The computer can operate software designed to record signals passed from the controller, and to drive the controller's output. Possible software includes Cambridge Electronic Design's (UK) SPIKE program. The software can be programmable and can record and analyze electrophysiological signals, as well as direct the controller 110 to deliver the broad spectrum of tunable electrical noise signals.

Patient monitor system. An optional patient monitor system (not shown) can also be used. The patient monitoring system can acquire, amplify, and filter physiological signals and then output them to the controller 110. The optional monitoring system can include a heart-rate monitor to collect electrocardiogram signals, and a muscle activity monitor to collect electromyography signals. The heart-rate monitor can include ECG electrodes coupled with an alternating current (AC) amplifier, while the muscle activity monitor can include EMG electrodes coupled with an AC amplifier. Other types of transducers may also be used. As described, all physiological signals obtained with the patient monitoring system are passed through an AC signal amplifier/conditioner. One possible amplifier/ conditioner is Model LP511 AC amplifier available from Grass Technologies, a subsidiary of Astro-Med, Inc., West Warwick, Rhode Island, USA.

Power System. All instruments are powered by a power supply or system 111. The power system 111 can include both external and internal portions, where the internal portion of the power system can include a battery (not shown), such as a lithium battery, while the external portion of the power system 111 can be plugged into a wall and can be used to recharge the battery as needed. The external portion of the power system 111 can transmit power to the noise generator 109 as directed by the controller 110 via RF signals/electromagnetic induction, or power can be transmitted to the noise generator 109 via the battery in the internal portion of the power system 111. Further, the external portion of the power system 111 can be used to recharge the battery in the internal portion of the power system 111.

Controller. A controller 110 can record electrical noise signal data as well as digital information from the optional patient monitor system, and can generate electrical noise signal and digital outputs simultaneously for real-time control of the noise generator 109 based on feedback received from the patient after transmission of the broad spectrum of tunable electrical noise signals. The controller 110 may have onboard memory to facilitate high speed data capture, independent waveform sample rates and on-line analysis. An exemplary controller 110 may be a POWER 1401 data-acquisition interface unit available from Cambridge Electronic Design (UK).

### Electrical Noise Signal Parameters

As discussed above, the broad spectrum of tunable electrical noise signals applied to the target neural tissue, non-neural tissue, or a combination thereof of the patient can include Gaussian noise, white noise, pink noise, red (Brownian) noise, grey noise, or any combination thereof in order to provide the desired therapy to the patient. The various types of electrical noise signals that can be utilized are discussed in more detail below.

First, in one embodiment, the broad spectrum of tunable electrical noise signals can include a Gaussian noise signal. A Gaussian noise signal includes a statistical noise having a probability density function (PDF) equal to that of the normal distribution, which is also known as the Gaussian distribution. In other words, the values that the noise can take on are Gaussian-distributed.

In another embodiment, the broad spectrum of tunable electrical noise signals can include a white noise signal. A white noise electrical signal refers to a signal having a flat frequency spectrum when plotted as a linear function of frequency and can thus be described as a random signal with a constant power spectral density (energy or power per Hz). In other words, the signal has equal power in any band of a given bandwidth (power spectral density) when the bandwidth is measured in Hz. For example, with a white noise signal, the range of frequencies between 40 Hz and 60 Hz contains the same amount of power as the range between 400 Hz and 420 Hz, since both intervals are 20 Hz wide.

In still another embodiment, a pink noise signal can also be utilized as part of the broad spectrum of tunable electrical noise signals delivered to the target neural tissue, non-neural tissue, or a combination thereof. A pink noise signal has a frequency spectrum that is linear in logarithmic space. As such, it has equal power in bands that are proportionally wide. This means that pink noise would have equal power in the frequency range from 40 to 60 Hz as in the frequency range from 4000 to 6000 Hz. Also called "1/f noise," pink noise is characterized by a frequency spectrum where the power spectral density (energy or power per Hz) is inversely proportional to the frequency of the signal. Since there are an infinite number of logarithmic bands at both the low frequency (DC) and high frequency ends of the spectrum, any finite energy spectrum must have less energy than pink noise at both ends. Pink noise is the only power-law spectral density that has this property: all steeper power-law spectra are finite if integrated to the high-frequency end, and all flatter power-law spectra are finite if integrated to the DC, low-frequency limit.

In yet another embodiment, a red of Brownian noise signal can be used. A red or Brownian noise signal is based on the concept of Brownian motion and can also be referred to as "random walk noise." Red or Brownian noise has a power spectral density that is inversely proportional to f², meaning it has more energy at lower frequencies, even more so than pink noise.

Meanwhile, in an additional embodiment, a grey noise signal can be utilized. A grey noise signal exhibits a frequency spectrum such that the power spectral density is equal at all frequencies.

Regardless of the particular type or combination of electrical noise signals utilized, the broad spectrum of electrical noise signals can be tuned, such that the energy contained within a particular frequency band, and for all frequency bands of energy delivered to the tissue, can be adjusted to best treat the patient. The broad spectrum of tunable electrical noise energy can be adjusted to deliver electrical noise with intensities ranging from about 0.01 volts (V) to about 200 V, such as from about 0.1 V to about 100 V, such as from about 0.5 V to about 50 V, for all or each frequency band included in the spectrum. The spectrum of electrical noise includes frequencies ranging from about 0.001 hertz (Hz) to about 500 kilohertz (kHz), such as from about 0.01 Hz to about 250 kHz, such as from about 0.05 Hz to about 200 kHz, and is composed of tunable frequency bands ranging from about 1 Hz to about 100 kHz, such as from about 5 Hz to about 75 kHz, such as from about 10 Hz to about 50 kHz.

### Method for Delivering a Broad Spectrum of Tunable Electrical Noise Signals

In addition to the systems discussed above, the present invention also encompasses a method for providing therapy to a patient that is customizable based on the particular circumstances present at the time the therapy is provided. For instance, after positioning one or more electrodes adjacent the target neural tissue, non-neural tissue, or a combination thereof (e.g., within or adjacent the patient's brain or spinal cord, a dorsal root ganglion, a sympathetic chain ganglion, or a peripheral nerve), the electrode(s) can be electrically connected to an implantable noise generator via a lead or to an external noise generator wirelessly. Then, a user interface and controller can be configured to deliver a broad spectrum of tunable electrical noise signals to provide therapy to the patient. The broad spectrum of electrical noise signals can be tuned, such that the energy contained within a particular frequency band, and for all frequency bands of energy delivered to the tissue, can be adjusted to best treat the patient. The broad spectrum of tunable electrical noise energy can be adjusted to deliver electrical noise with intensities ranging from about 0.01 volts (V) to about 200 V, such as from about 0.1 V to about 100 V, such as from about 0.5 V to about 50 V, for all or each frequency band included in the spectrum. The spectrum of electrical noise includes frequencies ranging from about 0.001 hertz (Hz) to about 500 kilohertz (kHz), such as from about 0.01 Hz to about 250 kHz, such as from about 0.05 Hz to about 200 kHz, and is composed of tunable frequency bands ranging from about 1 Hz to about 100 kHz, such as from about 5 Hz to about 75 kHz, such as from about 10 Hz to about 50 kHz.

After the broad spectrum of tunable electrical noise signals is delivered, patient feedback can be used to optimize the therapy provided to the patient. For example, in one particular embodiment, the broad spectrum of electrical noise signals can be tuned based on patient feedback by adjusting energy contained within a frequency band, while in another embodiment, the broad spectrum of electrical noise signals can be tuned based on patient feedback by adjusting a phase component of the broad spectrum. For example, one or more electrodes can be implanted, inserted percutaneously, or positioned transcutaneously such that the electrodes are nearby the target neural tissue, non-neural tissue and combination thereof as necessary to treat their disease or syndrome. A noise generator can then be instructed to deliver a broad spectrum of electrical noise signals through the one or more electrodes. The patient and/or caregiver can then program the optimal stimulation waveform by operating a controller. The controller can tune the waveform associated with the broad spectrum of electrical noise signals being delivered to the patient by adjusting energy levels within a particular frequency band, and for all frequency bands delivered, to best treat the patient. While the invention has been described in detail with respect to the specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims and any equivalents thereto.

## Claims

1. A system (50; 100; 300) for providing therapy to a patient, the system comprising:
an electrode (107; 107a; 107b);
a noise generator (109) coupled to the electrode (107; 107a; 107b); and
a controller (110);
wherein the controller (110) is adapted to instruct the noise generator (109) to deliver a broad spectrum of electrical noise signals to target neural tissue, non-neural tissue, or a combination thereof via the electrode (107; 107a; 107b), wherein the broad spectrum of electrical noise signals are tunable, **characterised in that** the controller (110) is configured to tune the broad spectrum of electrical noise signals to optimize the therapy provided to the patient based on feedback received from the patient.

2. The system (50; 100, 300) of claim 1, wherein the controller (110) tunes the broad spectrum of electrical noise signals by adjusting energy contained within a frequency band.

3. The system (50; 100, 300) of claim 1, wherein the controller (110) tunes the broad spectrum of electrical noise signals by adjusting a phase component of the broad spectrum of electrical noise signals.

4. The system (50; 100, 300) of claim 1, wherein the electrode (107; 107a; 107b) is percutaneous.

5. The system (50; 100, 300) of claim 1, wherein the electrode (107; 107a; 107b) is transcutaneous.

6. The system (50; 100, 300) of claim 1, wherein the electrode (107; 107a; 107b) is implantable.

7. The system (50; 100, 300) of any one of the preceding claims, wherein the noise generator (109) is implantable.

8. The system (50; 100, 300) of any one of the preceding claims, wherein the noise generator (109) is positioned external to the patient.

9. The system (50; 100, 300) of any one of the preceding claims, wherein the broad spectrum of electrical noise signals includes Gaussian noise, white noise, pink noise, Brownian noise, grey noise, or a combination thereof.

10. The system (50; 100, 300) of any one of the preceding claims, wherein only tuned electrical noise signals are delivered to the target neural tissue, non-neural tissue, or a combination thereof.

11. A system Z (50; 100; 300) of one of the preceding claims for providing therapy treating at least one of pain, an autonomic disorder, a sensory disorder, a motor disorder to a Patient.

12. A system (50; 100; 300) of one of the claims 1 - 10 for eliciting plastic changes in neural tissue, non-neural tissue, or a combination thereof to mitigate or abolish a pathophysiologic disease or syndrome.

13. A system (50; 100; 300) of one of the claims 1 - 10 for delivering electrical noise signals to target neural tissue, non-neural tissue, or a combination thereof, which is located within or adjacent a patient's brain or spinal cord, a dorsal root ganglion, a sympathetic chain ganglion, or a peripheral nerve.

## Patentansprüche

1. System (50; 100; 300) zum Bereitstellen einer Therapie für einen Patienten, wobei das System umfasst:
eine Elektrode (107; 107a; 107b);
einen Rauschgenerator (109), der mit der Elektrode (107; 107a; 107b) gekoppelt ist; und
einen Kontroller (110);
wobei der Kontroller (110) dazu angepasst ist, den Rauschgenerator (109) anzuweisen, über die Elektrode (107; 107a; 107b) ein breites Spektrum von elektrischen Rauschsignalen an neurales Zielgewebe, nicht-neurales Gewebe oder eine Kombination davon abzugeben,
wobei das breite Spektrum von elektrischen Rauschsignalen abstimmbar ist,
**dadurch gekennzeichnet, dass**
der Kontroller (110) dazu eingerichtet ist, das breite Spektrum von elektrischen Rauschsignalen zur Optimierung der für den Patienten bereitgestellten Therapie anhand eines von dem Patienten erhaltenen Feedbacks abzustimmen.

2. System nach Anspruch 1, wobei der Kontroller (110) das breite Spektrum von elektrischen Rauschsignalen durch Anpassen der Energie innerhalb eines Frequenzbandes abstimmt.

3. System (50; 100; 300) nach Anspruch 1, wobei der Kontroller (110) das breite Spektrum von elektrischen Rauschsignalen durch Anpassen einer Phasenkomponente des breiten Spektrums von elektrischen Rauschsignale abstimmt.

4. System (50; 100; 300) nach Anspruch 1, wobei die Elektrode (107; 107a; 107b) perkutan ist.

5. System (50; 100; 300) nach Anspruch 1, wobei die Elektrode (107; 107a; 107b) transkutan ist.

6. System (50; 100; 300) nach Anspruch 1, wobei die Elektrode (107; 107a; 107b) implantierbar ist.

7. System (50; 100; 300) nach einem der vorstehenden Ansprüche, wobei der Rauschgenerator (109) implantierbar ist.

8. System (50; 100; 300) nach einem der vorstehenden Ansprüche, wobei der Rauschgenerator (109) außerhalb des Patienten angeordnet ist.

9. System (50; 100; 300) nach einem der vorstehenden Ansprüche, wobei das breite Spektrum von elektrischen Rauschsignalen Gaußsches Rauschen, weißes Rauschen, rosa Rauschen, Brownsches Rauschen, graues Rauschen oder eine Kombination davon enthält.

10. System (50; 100; 300) nach einem der vorstehenden Ansprüche, wobei nur abgestimmte elektrische Rauschsignale an das neurale Zielgewebe, nicht neurale Gewebe oder eine Kombination davon abgegeben werden.

11. System (50; 100; 300) nach einem der vorstehenden Ansprüche zur Bereitstellung einer Therapie zur Behandlung von Schmerz, einer autonomen Störung, einer sensorischen Störung und/oder einer motorischen Störung bei einem Patienten.

12. System (50;100; 300) nach einem der Ansprüche 1-10 zum Hervorrufen plastischer Veränderungen in neuralem Gewebe, nicht-neuralem Gewebe oder einer Kombination, um eine pathophysiologische Krankheit oder ein pathophysiologisches Syndrom zu lindern.

13. System (50;100; 300) nach einem der Ansprüche 1-10 zum Abgeben von elektrischen Rauschsignalen an neurales Zielgewebe, nicht-neurales Gewebe oder eine Kombination davon, das in oder benachbart zu einem Gehirn, einem Rückenmark, einem Spinalganglion, einem sympathischem Grenzstrangganglion oder einem peripheren Nerv des Patienten lokalisiert ist.

## Revendications

1. Système (50 ; 100 ; 300) pour procurer une thérapie à un patient
une électrode (107 ; 107a ; 107b) ;
un générateur (109) de bruit couplé à l'électrode (107 ; 107a ; 107b) ; et
une unité (110) de commande ;
dans lequel l'unité (110) de commande est conçue pour donner instruction au générateur (109) de bruit de fournir un spectre large de signaux électriques de bruit à du tissu neural cible, du tissu non neural ou à leurs combinaisons par l'intermédiaire de l'électrode (107 ; 107a ; 107b), le spectre large de signaux électriques de bruit pouvant être accordé, **caractérisé en ce que** l'unité (110) de commande est configurée pour accorder le spectre large de signaux électriques de bruit, afin d'optimiser la thérapie procurée au patient sur la base d'une réaction reçue du patient.

2. Système (50 ; 100 ; 300) suivant la revendication 1, dans lequel l'unité (110) de commande accorde le spectre large de signaux électriques de bruit, en réglant de l'énergie contenue dans une bande de fréquence.

3. Système (50 ; 100 ; 300) suivant la revendication 1, dans lequel l'unité (110) de commande accorde le spectre large de signaux électriques de bruit, en réglant une composante de phase du spectre large de signaux électriques de bruit.

4. Système (50 ; 100 ; 300) suivant la revendication 1, dans lequel l'électrode (107, 107a, 107b) est percutanée.

5. Système (50 ; 100 ; 300) suivant la revendication 1, dans lequel l'électrode (107, 107a, 107b) est transcutanée.

6. Système (50 ; 100 ; 300) suivant la revendication 1, dans l'électrode (107, 107a, 107b) est implantable.

7. Système (50 ; 100 ; 300) suivant l'une quelconque des revendications précédentes, dans lequel le générateur (109) de bruit est implantable.

8. Système (50 ; 100 ; 300) suivant l'une quelconque des revendications précédentes, dans lequel le générateur (109) de bruit est placé à l'extérieur du patient.

9. Système (50 ; 100 ; 300) suivant l'une quelconque des revendications précédentes, dans lequel le spectre large de signaux électriques de bruit comprend du bruit gaussien, du bruit blanc, du bruit rose, du bruit brownien, du bruit gris ou leurs combinaisons.

10. Système (50 ; 100 ; 300) suivant l'une quelconque des revendications précédentes, dans lequel seulement des signaux électriques de bruit accordés sont envoyés au tissu neural cible, au tissu non-neural ou à leurs combinaisons.

11. Système (50 ; 100 ; 300) suivant l'une des revendications précédentes, pour procurer une thérapie traitant au moins l'un d'une douleur, d'un trouble neuro-végétatif, d'un trouble sensoriel, d'un trouble moteur chez un patient.

12. Système (50 ; 100 ; 300) suivant l'une des revendications 1 à 10, pour mettre en lumière des changements plastiques du tissu neural, du tissu non-neural ou de leurs combinaisons, afin d'adoucir ou d'abolir une maladie ou un syndrome pathophysiologique.

13. Système (50 ; 100 ; 300) suivant l'une des revendications 1 à 10, pour envoyer des signaux électriques de bruit à du tissu neural cible, du tissu non-neural ou à leurs combinaisons, qui est placé dans ou au voisinage du cerveau ou de la moelle épinière d'un patient, d'un ganglion de racine postérieure, d'un ganglion de chaîne sympathique ou d'un nerf périphérique.
